# EUROPEAN PATENT SPECIFICATION

(11) **EP 4 469 804 B1**
(45) Date of publication and mention of the grant of the patent: **14.10.2026**
(21) Application number: 22707601.5
(22) Date of filing: 31.01.2022
(51) Int. Cl.: G01N 33/68, G01N 33/543

(54) **METHOD FOR DETERMINATION OF MUTATED FORM OF MUCIN-1 PROTEIN IN A BIOLOGICAL SAMPLE**
VERFAHREN ZUR BESTIMMUNG EINER MUTIERTEN FORM DES MUCIN-1-PROTEINS IN EINER BIOLOGISCHEN PROBE
PROCÉDÉ DE DÉTERMINATION DE LA FORME MUTÉE DE LA PROTÉINE MUCINE-1 DANS UN ÉCHANTILLON BIOLOGIQUE

(30) Priority: 25.01.2022 CZ 20220034
(43) Date of publication of application: 04.12.2024
(73) Proprietor: Genespector S.r.o., 11000 Praha (CZ); Wake Forest University Health Sciences, Winston-Salem, NC 27157 (US)
(72) Inventor: KMOCH, Stanislav, 11000 Praha (CZ); ZIVNA, Martina, 12000 Praha (CZ); ZIVNY, Jan, 12000 Praha (CZ); TRESLOVA, Helena, 15000 Praha (CZ); BLEYER, Anthony, Winston-Salem, North Carolina 27106 (US); KIDD, Kendrah, Winston-Salem, North Carolina 27104 (US)
(74) Representative: Sedlák, Jirí
(86) International application number: PCT/CZ2022/050009
(87) International publication number: WO 2023/143650

(56) References cited:
- VYLET'AL PETR ET AL: "Plasma Mucin-1 (CA15-3) Levels in Autosomal Dominant Tubulointerstitial Kidney Disease due to MUC1 Mutations", AMERICAN JOURNAL OF NEPHROLOGY, vol. 52, no. 5, 1 January 2021 (2021-01-01), CH, pages 378 - 387, XP055960725, ISSN: 0250-8095, Retrieved from the Internet <URL:https://www.karger.com/Article/Pdf/515810> DOI: 10.1159/000515810
- PERSSON ET AL: "Antigens for the selection of pan-variable number of tandem repeats motif-specific human antibodies against Mucin-1", JOURNAL OF IMMUNOLOGICAL METHODS, ELSEVIER SCIENCE PUBLISHERS B.V.,AMSTERDAM, NL, vol. 316, no. 1-2, 30 October 2006 (2006-10-30), pages 116 - 124, XP005713788, ISSN: 0022-1759, DOI: 10.1016/J.JIM.2006.08.009
- KARSTEN U ET AL: "BINDING PATTERNS OF DTR-SPECIFIC ANTIBODIES REVEAL A GLYCOSYLATION-CONDITIONED TUMOR-SPECIFIC EPITOPE OF THE EPITHELIAL MUCIN (MUC1)", GLYCOBIOLOGY, OXFORD UNIVERSITY PRESS, US, vol. 14, no. 8, 1 August 2004 (2004-08-01), pages 681 - 692, XP008034908, ISSN: 0959-6658, DOI: 10.1093/GLYCOB/CWH090

## Description

### Technical field

The invention relates to immunoassays and biological material analysis, specifically to a method for determination of mutated form of mucin-4 protein in a biological sample and determination of position of its mutation and the relative length of individual forms of VNTRs.

### Background Art

Autosomal Dominant Tubulointerstitial Kidney Disease (ADTKD) is the second most common group of genetic kidney diseases after polycystosis. ADTKD is characterized by a dominant inheritance, normal urine and blood laboratory tests (except elevated serum creatinine and in some cases uric acid), tubular atrophy, interstitial fibrosis, and progressive renal failure requiring dialysis and transplantation. One category of ADTKD is a group associated with mutations in the *MUC1* gene (ADTKD-MUC1, formerly known as MCKD1). To date, all known cases of ADTKD-MUC1 are caused by frameshift mutations that result either in insertion of (3n+1) or deletions of (3n-1) nucleotides, in the variable number tandem repeats (VNTR) of region of the *MUC1* gene. *MUC1* is highly polymorphic due to the presence of a VNTR of 20-125 repeats, each containing 60 nucleotides, and also due to the occurrence of single nucleotide polymorphisms that affect *MUC1* mRNA splicing. Due to alternative splicing, *MUC1* pre-mRNA encodes several different proteins, including mucin-1 (MUC1). MUC1 is expressed in epithelial cells of a mucosal surface of several organs, especially the kidneys, lungs, breasts, intestines, esophagus, salivary glands, and pancreas. A key characteristic of MUC1 is a multiple O-glycosylation of the VNTR region, which plays a role as a signalling structure and gives this protein slimy properties.

The above-mentioned mutations in the form of insertions or deletions of DNA segments of different lengths lead to a frameshift during translation and, as a result, to the synthesis of a mutated variant of MUC1 (MUC1fs, frame-shifted). In a majority of the population affected by ADTKD-MUC1, this mutation occurs mostly within the VNTR region and only rarely before this region. Thus, in most cases, MUC1fs contains a partly natural, so-called wild-type, VNTR region at the N-terminus, i.e. the region encoded by the pre-mutated portion of the gene, and a mutated form of the VNTR region at the C-terminus, i.e. behind the mutation. The mutated form of the VNTR region contains 80 % fewer serine and threonine amino acids, which are normally extensively glycosylated, and is characterized by the presence of cysteine, leucine, histidine, and arginine amino acids. This sequence change determines that MUC1fs is an abnormal, non-naturally occurring, cysteine-rich, and highly basic protein that has completely unique biophysical properties, for example an isoelectric point >12. At the same time, due to the altered amino acid sequence, MUC1fs does not contain the usual structures at the C-terminus important for posttranslational modifications, cell signalling, proper anchoring in the cell membrane, and other molecular-biology events inherent to MUC1. As a result of these changes, MUC1fs accumulates within MUC1-producing cells and, in the case of the kidneys, causes their progressive failure. In patients diagnosed with ADTKD-MUC1, accumulation of MUC1fs has also been demonstrated in other tissues in which MUC1 is synthesized, such as breast tissue, lung epithelium, cutaneous sebaceous glands, or gastrointestinal mucosa, however, for unknown reasons without any clinical manifestation.

ADTKD-MUC1 therapy is currently limited to symptomatic treatment, such as supporting kidney function, dialysis, or kidney transplantation. Causal treatment is in an experimental stage, while three possible approaches are studied. First approach is a targeted blockade of the *MUC1* promoter, which prevents the synthesis of mutated MUC1fs, second approach is an application of a pharmaceutically active substance allowing MUC1fs to permeate into lysosomes where it degrades, where it degrades, thereby reducing the accumulated amount, and third approach is an application of a substance that increases the permeability of MUC1fs through the cell membrane into the extracellular space and its release it into the circulation, which again leads to a reduction of the amount accumulated inside cells, and thus relief to the kidney tissue.

Due to high content of guanosin and cytosin bases (85%), repetitive nature and the high variability caused by the different length of the VNTR region and the possibility of the mutation itself being anywhere within this region, it is impossible to detect ADTKD-MUC1 in a wide range of variants by standard genetic testing methods, and therefore it is very difficult to diagnose. Genetic, spectrometry-based assays (Blumenstiel B, DeFelice M, Birsoy O, et al. Development and Validation of a Mass Spectrometry-Based Assay for the Molecular Diagnosis of Mucin-1 Kidney Disease. J Mol Diagn 18:566-571, 2016) or amplicon sequencing (Wenzel A, Altmueller J, Ekici AB, et al. Single molecule real time sequencing in ADTKD-MUC1 allows complete assembly of the VNTR and exact positioning of causative mutations. Sci Rep 8:4170, 2018; Wang GQ, Rui HL, Dong HR, et al., SMRT sequencing revealed to be an effective method for ADTKD-MUC1 diagnosis through follow-up analysis of a Chinese family. Sci Rep 10, 2020) are known. However, these methods require complicated instrumental and can detect only a few specific variants of genetic mutations associated with ADTKD-MUC1. Furthermore, methods using immunohistochemical staining to detect the presence of MUC1fs in urinary cell swabs or in kidney biopsy are known (Zivna M, Kidd K, Pristoupilova A, et al. Noninvasive Immunohistochemical Diagnosis and Novel MUC1 Mutations Causing Autosomal Dominant Tubulointerstitial Kidney Disease. J Am Soc Nephrol 29:2418-2431, 2018; Knaup KX, Hackenbeck T, Popp B, et al., Biallelic Expression of Mucin-1 in Autosomal Dominant Tubulointerstitial Kidney Disease: Implications for Nongenetic Disease Recognition. J Am Soc Nephrol 29:2298-2309, 2018). However, these methods are associated with either highly invasive sampling or time consuming and, above all, do not allow the determination of free MUC1fs in blood plasma.

There are currently no known techniques for a quantitative determination of free MUC1fs in blood plasma. Such an assay would provide a number of advantages, as it would enable easy, fast, and minimally invasive differential diagnosis of hereditary chronic kidney disease as well as monitoring of a progression of this disease and an effectiveness of causal therapy by determining plasma levels of MUC1fs. In some types of tumors, there is a somatic mutation in the *MUC1* gene, due to which the tumor cells produce MUC1fs. Therefore, it is a suitable biomarker for the progression and the effectiveness of tumor therapy. Only an indirect method of diagnosing ADTKD-MUC1 by determination of non-mutated (wild-type) MUC1 in blood plasma is known, as in some patients suffering from ADTKD-MUC1, natural levels of non-mutated MUC1 are reduced in comparison with healthy population (Vyletal P, Kidd K, Ainsworth HC, Plasma Mucin-1 (CA15-3) Levels in Autosomal Dominant Tubulointerstitial Kidney Disease due to MUC1 Mutations Am J Nephrol 52:378-387, 2021). However, based on its principle, this method also cannot be used to monitor the progression of the disease, whether ADTKD-MUC1 or cancer, or the effectiveness of a therapy. Quantitative determination of free MUC1fs in plasma is further complicated by the highly variable length of the VNTR region, which varies significantly in the population, and the different location of the mutation in individual patients and their relatives. Thus, high demands are placed on determination techniques, such as the need to detect and determine an almost unlimited number of variants.

### Summary of Invention

The present invention discloses a method for determination of mutated form of mucin-1 protein in a biological sample and determination of position of its mutation and the relative length of individual forms of VNTRs. The method comprises steps of adding a biological sample to an immobilized antibody against the mutated form of variable number tandem repeats (VNTR) of mutated frame-shifted variant of mucin-1 protein (MUC1fs), adding an antibody against the wild-type form of VNTR MUC1fs to a portion of this mixture and performing a quantitative analysis, and adding an antibody against the mutated form of VNTR MUC1fs to the remainder of the mixture of the biological sample with the immobilized antibody against the mutated form of VNTR MUC1fs and performing quantitative analysis by comparing normalized signals. The present invention overcomes the aforementioned shortcomings by providing a quantitative determination of free MUC1fs in biological samples, for example blood plasma, using a method that is easy to perform, does not require specialized instrumentation, is feasible in any biochemical laboratory, and allows to determine MUC1fs regardless of VNTR region length or the location of the mutation in a specific patient.

The antibody against the mutated form of VNTR MUC1fs is recognized by amino acid sequence of a specific epitope SEQ ID NO. 1: SPRCHLGPGHQAGPGLHRPP. The antibody against the wild-type form of VNTR MUC1fs is recognized by amino acid sequence of a specific epitope SEQ ID NO.2: APDTRP. The antibody is immobilized on a support for example in a form of a gel or beads based on polymeric or magnetic material. The immobilized antibody is then incubated with a test sample of biological material, for example blood plasma, and the material is divided into two parts for the following quantitative analysis.

In the first part of the sample the quantitative analysis is performed using an antibody against the wild-type form of VNTR MUC1fs carrying a structure selected from the group consisting of horseradish peroxidase (HRP) enzyme with subsequent addition of a mixture of reactive dye and hydrogen peroxide and measurement of a change in absorbance of the solution or fluorescent label with a subsequent measurement of a change in fluorescence value or mass label with a subsequent measurement of a change in mass by mass spectrometry.

In the second part of the sample the quantitative analysis is performed using an antibody against the mutated form of VNTR MUC1fs carrying a structure selected from the group consisting of HRP enzyme with subsequent addition of a mixture of reactive dye and hydrogen peroxide and measurement of a change in absorbance of the solution or fluorescent label with a subsequent measurement of a change in fluorescence value or mass label with a subsequent measurement of a change in mass by mass spectrometry.

In another preferred embodiment, the quantitative analysis is performed in the first part of the sample using an antibody against the wild-type form of VNTR MUC1fs, while the antibody against the antibody is carrying a structure selected from the group consisting of HRP enzyme with subsequent addition of a mixture of reactive dye and hydrogen peroxide and measurement of a change in absorbance of the solution or fluorescent label with a subsequent measurement of a change in fluorescence value or mass label with a subsequent measurement of a change in mass by mass spectrometry. While the quantitative analysis is performed in the second part of the sample using an antibody against the antibody against the mutated form of VNTR MUC1fs, while the antibody against the antibody is carrying a structure selected from the group consisting of HRP enzyme with subsequent addition of a mixture of reactive dye and hydrogen peroxide and measurement of a change in absorbance of the solution or fluorescent label with a subsequent measurement of a change in fluorescence value or mass label with a subsequent measurement of a change in mass by mass spectrometry.

For determination of the concentration of MUC1fs in the sample, determination of the VNTR length, and determination of the mutated position, a length of the mutated region of VNTR MUC1fs in the sample is determined using appropriate calibration curve obtained by analysing a standard containing at least one epitope of the mutated form of VNTR MUC1fs, and a length of the wild-type region of VNTR MUC1 in the sample is determined using an appropriate calibration curve obtained by analysing a standard containing at least one epitope of the wild-type form of VNTR MUC1fs.

Inventive and non-obvious part of the invention is the parallel use of two different antibodies, one against the wild-type VNTR MUC1fs and one against the mutated VNTR MUC1fs. After normalization and quantification of the results using the modified-BSA standard, it is not only possible to determine the concentration of MUC1fs in a biological sample in any variant, i.e. regardless of VNTR length or mutation position, but it is also possible to determine the VNTR length and mutation position in the variant present in a specific sample. In general, four types of results can be obtained:
1. Low concentration of anti-wt-VNTR and low concentration of anti-fs-VNTR
2. High concentration of anti-wt-VNTR and low concentration of anti-fs-VNTR
3. Low concentration of anti-wt-VNTR and high concentration of anti-fs-VNTR
4. High concentration of anti-wt-VNTR and high concentration of anti-fs-VNTR

The type 1 outcome represents samples from healthy individuals who do not have a mutation in the *MUC1* gene. In exceptional cases, this may be a false negative result in patients with a very short VNTR region or a mutation very close to the C-terminus. In these cases, MUC1fs immobilization fails due to insufficient binding site for the immobilized antibody against the mutated VNTR MUC1fs. The type 2 outcome is observed in patients with a mutation near the C-terminus, the type 3 outcome in patients with a mutation near the N-terminus, and the type 4 outcome in patients with a mutation located in the middle of the VNTR region.

The present invention provides a method for monitoring the values or changes in the values of said biomarker in the form of MUC1fs, thus enabling monitoring of the progression of the disease or the effectiveness of its therapy.

### Brief Description of Drawings

Fig. 1

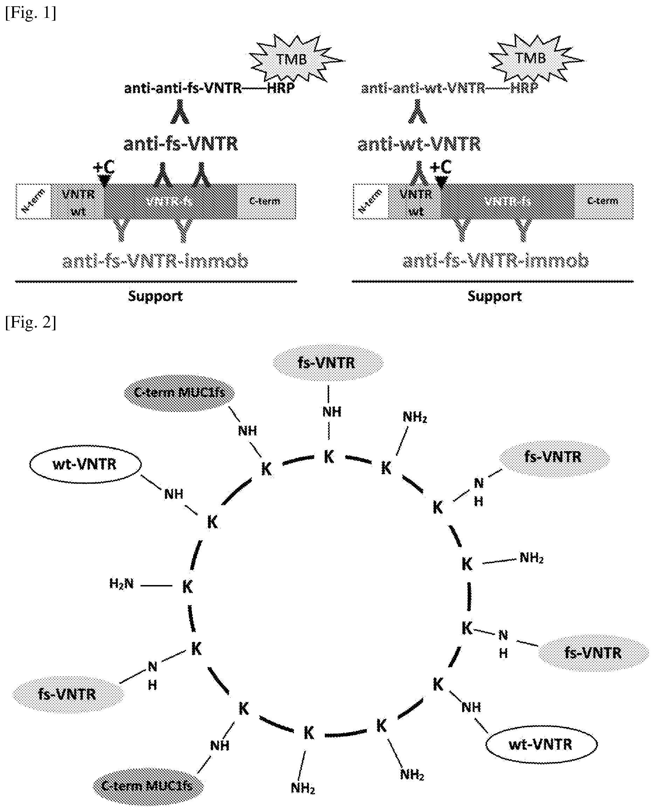

   [Fig.1] schematically shows a system for determining the plasma concentration of MUC1fs. The left diagram shows the determination of the length of fs-VNTR, the right diagram shows the determination of the length of wt-VNTR. The "+C" mark indicates the position of the mutation.
Fig. 2
   [Fig.2] schematically shows a standard based on modified BSA. The letters K represent chains of lysine amino acid.
Fig. 3

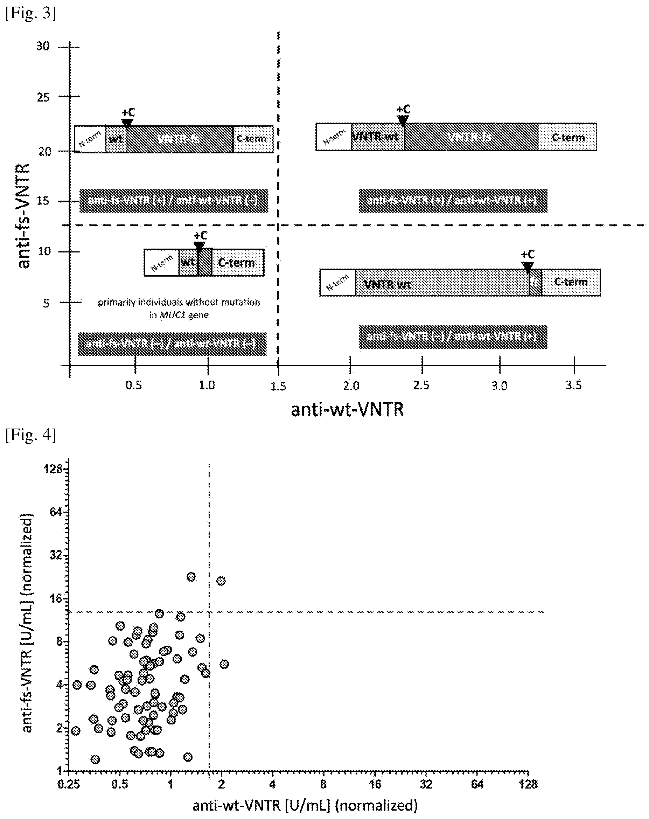

   [Fig. 3] schematically shows the relationship between the measured normalized anti-wt-VNTR and anti-fs-VNTR concentrations, the length of the VNTR region, and the mutation position.
Fig. 4
   [Fig. 4] shows a graph of measured anti-wt-VNTR and anti-fs-VNTR concentrations in a control group of healthy individuals. Each point represents one person.
Fig. 5

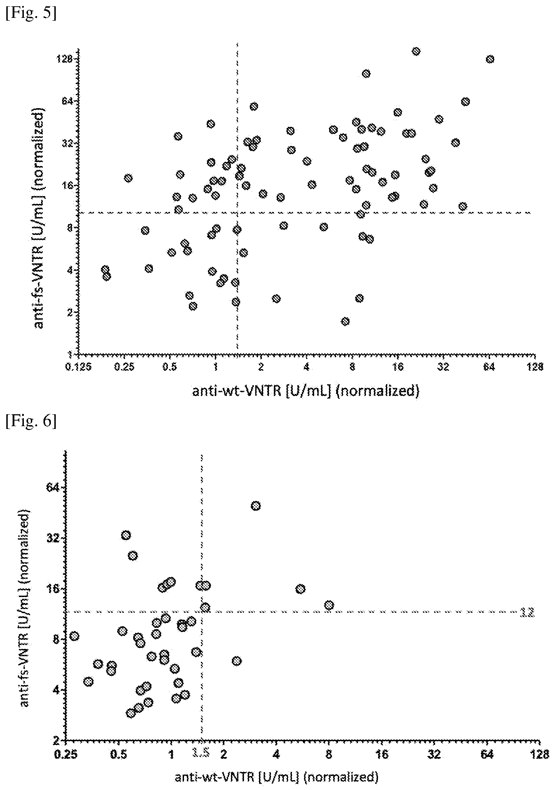

   [Fig. 5] shows a graph of measured anti-wt-VNTR and anti-fs-VNTR concentrations in a group of patients with ADTKD-MUC1. Each point represents one person.
Fig. 6
   [Fig. 6] shows a graph of measured anti-wt-VNTR and anti-fs-VNTR concentrations in a group of individuals unaffected by ADTKD-MUC1. Each point represents one person. The twelve individuals showing the presence of various forms of MUC1fs were subsequently diagnosed with cancer.
Fig. 7

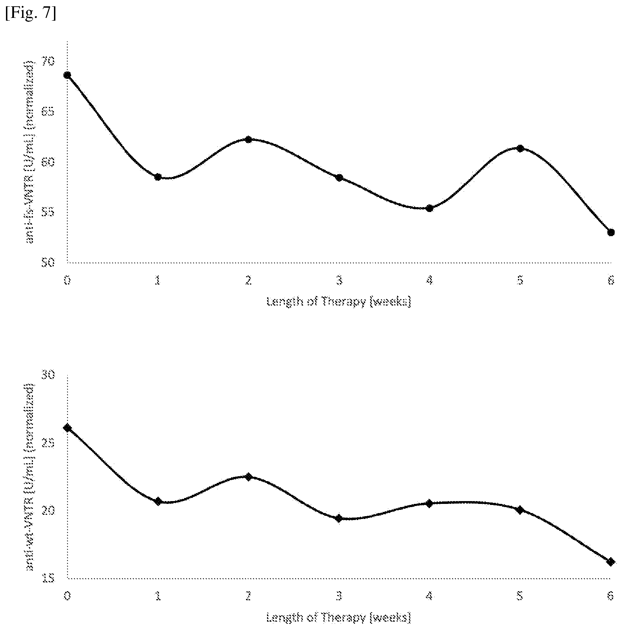

   [Fig. 7] shows graphs of measured anti-wt-VNTR and anti-fs-VNTR concentrations plotted against time in a patient with ADTKD-MUC1 undergoing an experimental treatment with MUC1 promoter blockade. Thus, it illustrates the ability of the invention to monitor the development of plasma concentration of MUC1fs during a therapy.
Fig. 8

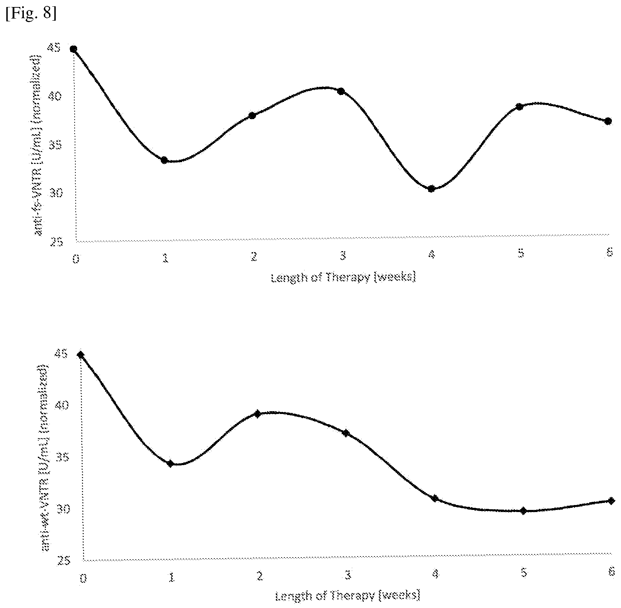

   [Fig. 8] shows graphs of measured anti-wt-VNTR and anti-fs-VNTR concentrations plotted against time in a patient with ADTKD-MUC1 undergoing an experimental treatment with a substance that promotes MUC1fs permeation into lysosomes. Thus, it illustrates the ability of the invention to monitor the development of plasma concentration of MUC1fs during a therapy.
Fig. 9

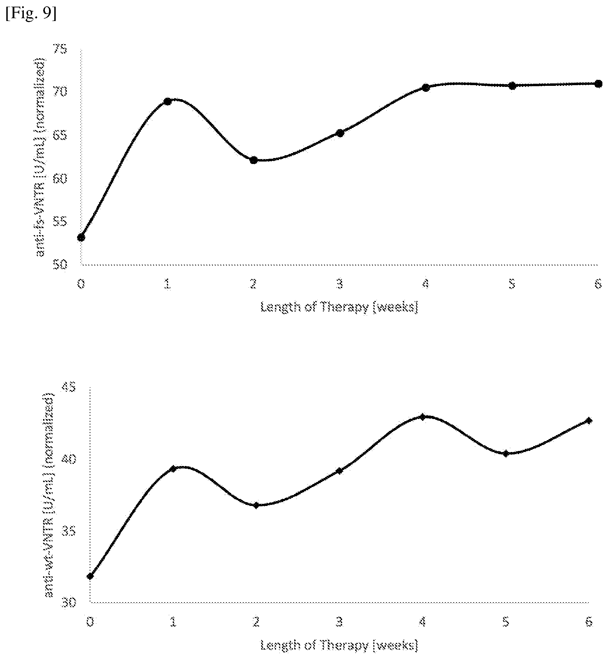

   [Fig. 9] shows graphs of measured anti-wt-VNTR and anti-fs-VNTR concentrations plotted against time in a patient with ADTKD-MUC1 undergoing an experimental treatment with a substance that promotes MUC1fs permeation through the cell membrane into the extracellular space. Thus, it illustrates the ability of the invention to monitor the development of plasma concentration of MUC1fs during a therapy.
Fig. 10

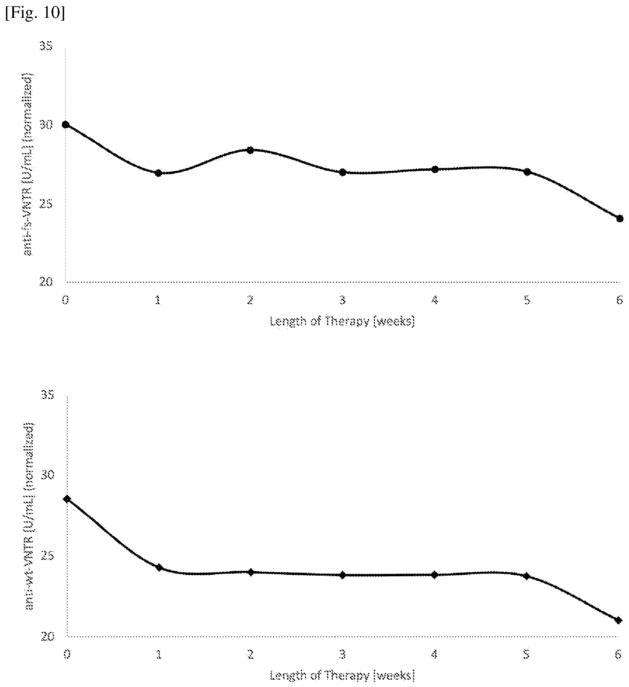

   [Fig. 10] shows graphs of measured anti-wt-VNTR and anti-fs-VNTR concentrations plotted against time in a patient with ovarian cancer and undergoing cytostatic treatment. Thus, it illustrates the ability of the invention to monitor the development of plasma concentration of MUC1fs during a therapy.

### Description of Embodiments

The references to the methods of treatment by therapy or surgery or in vivo diagnosis methods in examples 2, 3, 4 and 5 of this description are to be interpreted as referenced to compounds, pharmaceutical compositions and medicaments of the present invention for use in those methods.

### Example 1

Example 1 describes the determination of the concentration of MUC1fs in a blood plasma sample using a method according to the invention.

Into wells of two microtiter plates containing antibody-immobilization resin, 100 µL of phosphate buffer solution with 0.15 M NaCl (Phosphate Buffered Saline, PBS) containing antibody against the mutated form of VNTR MUC1fs (amino acid sequence of the specific epitope that the antibody recognizes: SEQ ID NO. 1: SPRCHLGPGHQAGPGLHRPP) at a concentration of 5 µg/mL is added and the plates are incubated overnight at 4 °C. After aspirating and washing the wells three times with PBS washing solution containing 0.05 % of polysorbate 20, the wells are filled with blocking solution, i.e. 1 % solution of unmodified BSA in PBS containing 0.05 % of polysorbate 20, and the plates are left for one hour at laboratory temperature. After aspirating the blocking solution, the plate is washed once with the washing solution. A standard in a serial dilution (2x) is added to a portion of the wells of each plate, starting at 1:32 for anti-wt-VNTR MUC1fs antibody detection (Plate 1) and 1:256 for anti-fs-VNTR MUC1fs antibody detection (Plate 2). A mixture containing a blood plasma sample obtained by centrifugation of collected peripheral venous blood twice diluted with PBS, 1 % of unmodified BSA, and 0.05 % of polysorbate 20 is added to the other wells and the plates are incubated for two hours at laboratory temperature. After that, the plates are aspirated and washed with the washing solution three times. Mouse monoclonal antibody against the wild-type of VNTR MUC1fs (amino acid sequence of the specific epitope that the antibody recognizes: SEQ ID NO. 2: APDTRP) at a dilution of 1:250 is added to Plate 1. After 2 hours of incubation at laboratory temperature, the plate is washed three times with the washing solution and an antibody against the mouse antibody carrying HRP at a dilution of 1:5,000 is added. The plate is incubated with this antibody for 1 hour at laboratory temperature. Rabbit antibody against the mutated form of VNTR MUC1fs (amino acid sequence of the specific epitope that the antibody recognizes: SEQ ID NO. 1: SPRCHLGPGHQAGPGLHRPP) at a dilution of 1:1,000 is added to Plate 2. After 2 hours of incubation at laboratory temperature, the plate is washed three times with the washing solution and an antibody against the rabbit antibody carrying HRP at a dilution of 1:5,000 is added. The plate is incubated with this antibody for 1 hour at laboratory temperature. Subsequently, both plates are aspirated and washed three times with PBS solution. Then, 100 µL of a standard solution containing tetramethylbenzidine (TMB) and hydrogen peroxide is added to both plates. The colour-change reaction is terminated after 15 minutes by an addition of 1 M sulfuric acid. Absorbance of the solutions in both individual plates is measured spectrophotometrically at 450 nm. Alternatively, directly labelled detection antibodies or directly labelled antibodies against anti-wt-VNTR and anti-fs-VNTR antibodies carrying specific fluorophores or mass labels attached to their molecules are used and quantified fluorimetrically or by mass spectrometry. To determine the concentration of MUC1fs in the plasma sample, a calibration curve is further prepared according to the abovementioned procedure using a modified-BSA standard at various dilutions. A 16-fold dilution represents 100 U/mL of repeats of the wild-type VNTR MUC1fs and a 256-fold dilution represents 100 U/mL of repeats of the mutated VNTR MUC1fs. The measured concentrations of the real samples are normalized to a normalization sample with the determined concentrations of 40 U/mL of anti-fs-VNTR and 6 U/mL of anti-wt-VNTR.

### Example 2

Example 2 demonstrates monitoring of the efficacy of an experimental treatment by blocking the *MUC1* promoter in a patient suffering from ADTKD-MUC1 using the method of the invention.

A peripheral venous blood sample, from which plasma is separated by centrifugation, is taken from the patient at weekly intervals for six weeks. The presence of MUC1fs in plasma is then determined by the method described in Example 1. The measured normalized values of anti-fs-VNTR and anti-wt-VNTR concentrations and their development over time are summarized in the following table:

| **Length of Therapy** | **anti-fs-VNTR [U/mL]** | **anti**-**wt-VNTR [U/mL]** |
|---|---|---|
| beginning | 68.678 | 26.151 |
| 1 week | 58.537 | 20.707 |
| 2 weeks | 62.265 | 22.514 |
| 3 weeks | 58.472 | 19.460 |
| 4 weeks | 55.458 | 20.559 |
| 5 weeks | 61.368 | 20.089 |
| 6 weeks | 53.037 | 16.252 |

### Example 3

Example 3 demonstrates monitoring of the efficacy of an experimental treatment with a substance that promotes a permeation of MUC1fs into lysosomes in a patient suffering from ADTKD-MUC1 using the method of the invention.

A peripheral venous blood sample, from which plasma is separated by centrifugation, is taken from the patient at weekly intervals for six weeks. The presence of MUC1fs in plasma is then determined by the method described in Example 1. The measured normalized values of anti-fs-VNTR and anti-wt-VNTR concentrations and their development over time are summarized in the following table:

| **Length of Therapy** | **anti-fs-VNTR [U/mL]** | **anti-wt-VNTR [U/mL]** |
|---|---|---|
| beginning | 44.828 | 44.885 |
| 1 week | 33.292 | 34.268 |
| 2 weeks | 37.762 | 38.967 |
| 3 weeks | 40.168 | 37.007 |
| 4 weeks | 29.970 | 30.542 |
| 5 weeks | 38.300 | 29.224 |
| 6 weeks | 36.724 | 30.101 |

### Example 4

Example 4 demonstrates monitoring of the efficacy of an experimental treatment with a substance that increases a permeation of MUC1fs through the cell membrane into the extracellular space in a patient suffering from ADTKD-MUC1 using the method of the invention.

A peripheral venous blood sample, from which plasma is separated by centrifugation, is taken from the patient at weekly intervals for six weeks. The presence of MUC1fs in plasma is then determined by the method described in Example 1. The measured normalized values of anti-fs-VNTR and anti-wt-VNTR concentrations and their development over time are summarized in the following table:

| **Length of Therapy** | **anti-fs-VNTR [U/mL]** | **anti-wt-VNTR [U/mL]** |
|---|---|---|
| beginning | 53.235 | 31.845 |
| 1 week | 68.964 | 39.332 |
| 2 weeks | 62.187 | 36.815 |
| 3 weeks | 65.315 | 39.201 |
| 4 weeks | 70.552 | 42.963 |
| 5 weeks | 70.785 | 40.405 |
| 6 weeks | 71.025 | 42.712 |

### Example 5

Example 5 demonstrates monitoring of the efficacy of a cytostatic treatment in a patient suffering from ovarian cancer using the method of the invention.

A peripheral venous blood sample, from which plasma is separated by centrifugation, is taken from the patient at weekly intervals for six weeks. The presence of MUC1fs in plasma is then determined by the method described in Example 1. The measured normalized values of anti-fs-VNTR and anti-wt-VNTR concentrations and their development over time are summarized in the following table:

| **Length of Therapy** | **anti-fs-VNTR [U/mL]** | **anti-wt-VNTR [U/mL]** |
|---|---|---|
| beginning | 30.036 | 28.552 |
| 1 week | 26.966 | 24.295 |
| 2 weeks | 28.413 | 24.011 |
| 3 weeks | 26.997 | 23.829 |
| 4 weeks | 27.183 | 23.840 |
| 5 weeks | 27.028 | 23.761 |
| 6 weeks | 24.074 | 21.036 |

## Claims

1. Method for determination of mutated form of mucin-1 protein in a biological sample and determination of position of its mutation and the relative length of individual forms of VNTRs, **characterized in that** it comprises steps of adding a biological sample to an immobilized antibody against the mutated form of variable number tandem repeats (VNTR) of mutated frame-shifted variant of mucin-1 protein (MUC1fs), adding an antibody against the wild-type form of VNTR MUC1fs to a portion of this mixture and performing a quantitative analysis, and adding an antibody against the mutated form of VNTR MUC1fs to the remainder of the mixture of the biological sample with the immobilized antibody against the mutated form of VNTR MUC1fs and performing quantitative analysis by comparing normalized signals.

2. Method according to claim 1, **characterized in that** the amino acid sequence of a specific epitope that the antibody against the mutated form of VNTR MUC1fs recognizes is SEQ ID NO. 1.

3. Method according to claim 1 or 2, **characterized in that** the amino acid sequence of a specific epitope that the antibody against the wild-type form of VNTR MUC1fs recognizes is SEQ ID NO. 2.

4. Method according to any of claims 1 to 3, **characterized in that** the quantitative analysis is performed using an antibody against the wild-type form of VNTR MUC1fs carrying a structure selected from the group consisting of horseradish peroxidase (HRP) enzyme with subsequent addition of a mixture of reactive dye and hydrogen peroxide and measurement of a change in absorbance of the solution or fluorescent label with a subsequent measurement of a change in fluorescence value or mass label with a subsequent measurement of a change in mass by mass spectrometry.

5. Method according to any of claims 1 to 4, **characterized in that** the quantitative analysis is performed using an antibody against the mutated form of VNTR MUC1fs carrying a structure selected from the group consisting of HRP enzyme with subsequent addition of a mixture of reactive dye and hydrogen peroxide and measurement of a change in absorbance of the solution or fluorescent label with a subsequent measurement of a change in fluorescence value or mass label with a subsequent measurement of a change in mass by mass spectrometry.

6. Method according to any of claims 1 to 3, **characterized in that** the quantitative analysis is performed using an antibody against the antibody against the wild-type form of VNTR MUC1fs, while the antibody against the antibody is carrying a structure selected from the group consisting of HRP enzyme with subsequent addition of a mixture of reactive dye and hydrogen peroxide and measurement of a change in absorbance of the solution or fluorescent label with a subsequent measurement of a change in fluorescence value or mass label with a subsequent measurement of a change in mass by mass spectrometry.

7. Method according to any of claims 1 to 4 or 6, **characterized in that** the quantitative analysis is performed using an antibody against the antibody against the mutated form of VNTR MUC1fs, while the antibody against the antibody is carrying a structure selected from the group consisting of HRP enzyme with subsequent addition of a mixture of reactive dye and hydrogen peroxide and measurement of a change in absorbance of the solution or fluorescent label with a subsequent measurement of a change in fluorescence value or mass label with a subsequent measurement of a change in mass by mass spectrometry.

8. Method according to any of claims 1 to 7, **characterized in that** for determination of the concentration of MUC1fs in the sample, determination of the VNTR length, and determination of the mutated position, a length of the mutated region of VNTR MUC1fs in the sample is determined using appropriate calibration curve obtained by analysing a standard containing at least one epitope of the mutated form of VNTR MUC1fs, and a length of the wild-type region of VNTR MUC1 in the sample is determined using an appropriate calibration curve obtained by analysing a standard containing at least one epitope of the wild-type form of VNTR MUC1fs.

## Patentansprüche

1. Verfahren zur Bestimmung der mutierten Form des Mucin-1-Proteins in einer biologischen Probe und zur Bestimmung der Position seiner Mutation und der relativen Länge einzelner Formen von VNTRs, **dadurch gekennzeichnet, dass** es Schritte der Zugabe einer biologischen Probe zu einem immobilisierten Antikörper gegen die mutierte Form von Tandem-Wiederholungen mit variabler Anzahl (VNTR) der mutierten Variante des Mucin-1-Proteins (MUC1fs) mit Leserasterverschiebung, Zugabe eines Antikörpers gegen die Wild-type-Form von VNTR MUC1fs zu einem Teil dieser Mischung und Durchführung einer quantitativen Analyse, und Zugabe eines Antikörpers gegen die mutierte Form von VNTR MUC1fs zum Rest der Mischung aus der biologischen Probe und dem immobilisierten Antikörper gegen die mutierte Form von VNTR MUC1fs und Durchführung einer quantitativen Analyse durch Vergleich normalisierter Signale umfasst.

2. Verfahren nach Anspruch 1, **dadurch gekennzeichnet, dass** die Aminosäuresequenz eines spezifischen Epitops, das der Antikörper gegen die mutierte Form von VNTR MUC1fs erkennt, folgende SEQ ID NO. hat: 1.

3. Verfahren nach Anspruch 1 oder 2, **dadurch gekennzeichnet, dass** die Aminosäuresequenz eines spezifischen Epitops, das der Antikörper gegen die Wild-type-Form von VNTR MUC1fs erkennt, folgende SEQ ID NO. hat: 2.

4. Verfahren nach einem der Ansprüche 1 bis 3, **dadurch gekennzeichnet, dass** die quantitative Analyse unter Verwendung eines Antikörpers gegen die Wild-type-Form von VNTR MUC1fs, der eine Struktur trägt, die aus der Gruppe bestehend aus dem Enzym Meerrettichperoxidase (HRP) ausgewählt ist, mit anschließender Zugabe einer Mischung aus reaktivem Farbstoff und Wasserstoffperoxid und Messung einer Änderung der Absorption der Lösung oder Fluoreszenzmarkierung mit anschließender Messung einer Änderung des Fluoreszenzwertes oder Massenmarkierung mit anschließender Messung einer Massenänderung mittels Massenspektrometrie durchgeführt wird.

5. Verfahren nach einem der Ansprüche 1 bis 4, **dadurch gekennzeichnet, dass** die quantitative Analyse unter Verwendung eines Antikörpers gegen die mutierte Form von VNTR MUC1fs, der eine Struktur trägt, die aus der Gruppe bestehend aus HRP-Enzym ausgewählt ist, und anschließender Zugabe einer Mischung aus reaktivem Farbstoff und Wasserstoffperoxid und Messung einer Änderung der Absorption der Lösung oder Fluoreszenzmarkierung mit anschließender Messung einer Änderung des Fluoreszenzwertes oder Massenmarkierung mit anschließender Messung einer Massenänderung mittels Massenspektrometrie durchgeführt wird.

6. Verfahren nach einem der Ansprüche 1 bis 3, **dadurch gekennzeichnet, dass** die quantitative Analyse unter Verwendung eines Antikörpers gegen die Wild-type-Form von VNTR MUC1fs durchgeführt wird, wobei der Antikörper gegen den Antikörper eine Struktur trägt, die aus der Gruppe bestehend aus HRP-Enzym ausgewählt ist, mit anschließender Zugabe einer Mischung aus reaktivem Farbstoff und Wasserstoffperoxid und Messung einer Änderung der Absorption der Lösung oder Fluoreszenzmarkierung mit anschließender Messung einer Änderung des Fluoreszenzwertes oder Massenmarkierung mit anschließender Messung einer Massenänderung mittels Massenspektrometrie.

7. Verfahren nach einem der Ansprüche 1 bis 4 oder 6, **dadurch gekennzeichnet, dass** die quantitative Analyse unter Verwendung eines Antikörpers gegen den Antikörper gegen die mutierte Form von VNTR MUC1fs durchgeführt wird, wobei der Antikörper gegen den Antikörper eine Struktur trägt, die aus der Gruppe bestehend aus HRP-Enzym ausgewählt ist, mit anschließender Zugabe einer Mischung aus reaktivem Farbstoff und Wasserstoffperoxid und Messung einer Änderung der Absorption der Lösung oder Fluoreszenzmarkierung mit anschließender Messung einer Änderung des Fluoreszenzwertes oder Massenmarkierung mit anschließender Messung einer Massenänderung mittels Massenspektrometrie.

8. Verfahren nach einem der Ansprüche 1 bis 7, **dadurch gekennzeichnet, dass** zur Bestimmung der Konzentration von MUC1fs in der Probe, zur Bestimmung der VNTR-Länge und zur Bestimmung der mutierten Position die Länge der mutierten Region von VNTR MUC1fs in der Probe unter Verwendung einer geeigneten Kalibrierkurve bestimmt wird, die durch Analyse eines Standards erhalten wurde, der mindestens ein Epitop der mutierten Form von VNTR MUC1fs enthält, und die Länge der Wild-type-Region von VNTR MUC1 in der Probe unter Verwendung einer geeigneten Kalibrierkurve bestimmt wird, die durch Analyse eines Standards erhalten wurde, der mindestens ein Epitop der Wild-type-Form von VNTR MUC1fs enthält.

## Revendications

1. Méthode de détermination de la forme mutée de la protéine mucine-1 dans un échantillon biologique et de détermination de la position de sa mutation et de la longueur relative des différentes formes de VNTR, **caractérisée en ce qu'**elle comprend les étapes consistant à ajouter un échantillon biologique à un anticorps immobilisé dirigé contre la forme mutée des répétitions en tandem à nombre variable (VNTR) de la variante mutée à décalage du cadre de lecture de la protéine mucine-1 (MUC1fs), à ajouter un anticorps dirigé contre la forme de type sauvage de VNTR MUC1fs à une partie de ce mélange et à effectuer une analyse quantitative, puis à ajouter un anticorps dirigé contre la forme mutée de VNTR MUC1fs à la partie restante du mélange de l'échantillon biologique avec l'anticorps immobilisé dirigé contre la forme mutée de VNTR MUC1fs et à effectuer une analyse quantitative en comparant des signaux normalisés.

2. Méthode selon la revendication 1, **caractérisée en ce que** la séquence d'acides aminés d'un épitope spécifique reconnu par l'anticorps dirigé contre la forme mutée de VNTR MUC1fs est la SEQ ID NO. 1.

3. Méthode selon la revendication 1 ou 2, **caractérisée en ce que** la séquence d'acides aminés d'un épitope spécifique reconnu par l'anticorps dirigé contre la forme de type sauvage de VNTR MUC1fs est la SEQ ID NO. 2.

4. Méthode selon l'une quelconque des revendications 1 à 3, **caractérisée en ce que** l'analyse quantitative est réalisée en utilisant un anticorps dirigé contre la forme de type sauvage de VNTR MUC1fs portant une structure sélectionnée dans le groupe constitué de l'enzyme peroxydase de raifort (HRP), suivie de l'addition d'un mélange de colorant réactif et de peroxyde d'hydrogène et de la mesure d'un changement de l'absorbance de la solution, ou d'une étiquette fluorescente suivi de la mesure d'un changement de valeur de fluorescence ou d'une étiquette de masse avec une mesure subséquente d'un changement de masse par spectrométrie de masse.

5. Méthode selon l'une quelconque des revendications 1 à 4, **caractérisée en ce que** l'analyse quantitative est réalisée en utilisant un anticorps dirigé contre la forme mutée de VNTR MUC1fs portant une structure sélectionnée dans le groupe constitué de l'enzyme HRP, suivi de l'addition d'un mélange de colorant réactif et de peroxyde d'hydrogène et de la mesure d'un changement de l'absorbance de la solution, ou d'une étiquette fluorescente suivi de la mesure d'un changement de valeur de fluorescence, ou d'une étiquette de masse suivi de la mesure d'un changement de masse par spectrométrie de masse.

6. Méthode selon l'une quelconque des revendications 1 à 3, **caractérisée en ce que** l'analyse quantitative est réalisée en utilisant un anticorps dirigé contre l'anticorps dirigé contre la forme de type sauvage de VNTR MUC1fs, tandis que l'anticorps contre l'anticorps porte une structure sélectionnée dans le groupe constitué par l'enzyme HRP avec l'addition subséquente d'un mélange de colorant réactif et de peroxyde d'hydrogène et mesure d'un changement de l'absorbance de la solution ou d'une étiquette fluorescente avec mesure subséquente d'un changement de la valeur de fluorescence ou d'une étiquette de masse avec une mesure subséquente d'un changement de masse par spectrométrie de masse.

7. Méthode selon l'une quelconque des revendications 1 à 4 ou 6, **caractérisée en ce que** l'analyse quantitative est réalisée en utilisant un anticorps dirigé contre l'anticorps dirigé contre la forme mutée de VNTR MUC1fs, tandis que l'anticorps dirigé contre l'anticorps porte une structure sélectionnée dans le groupe constitué par l'enzyme HRP avec l'addition subséquente d'un mélange de colorant réactif et de peroxyde d'hydrogène et mesure d'un changement de l'absorbance de la solution ou d'une étiquette fluorescente avec mesure subséquente d'un changement de valeur de fluorescence ou d'une étiquette de masse avec une mesure subséquente d'un changement de masse par spectrométrie de masse.

8. Méthode selon l'une quelconque des revendications 1 à 7, **caractérisée en ce que** pour la détermination de la concentration de MUC1fs dans l'échantillon, la détermination de la longueur de VNTR et la détermination de la position mutée, une longueur de la région mutée de VNTR MUC1fs dans l'échantillon est déterminée en utilisant une courbe d'étalonnage appropriée obtenue par l'analyse d'un standard contenant au moins un épitope de la forme mutée de VNTR MUC1fs, et une longueur de la région de type sauvage de VNTR MUC1 dans l'échantillon est déterminée en utilisant une courbe d'étalonnage appropriée obtenue par l'analyse d'un standard contenant au moins un épitope de la forme de type sauvage de VNTR MUC1fs.
